# EUROPEAN PATENT APPLICATION

(11) **EP 3 309 140 A1**
(43) Date of publication of application: **18.04.2018**
(21) Application number: 17204288.9
(22) Date of filing: 06.06.2013
(51) Int. Cl.: C07C 21/18

(54) **COMPOSITIONS COMPRISING FLUOROOLEFINS**

(30) Priority: 06.06.2012 US 201261656125 P
(62) Divisional of application: 13800895.8
(71) Applicant: The Chemours Company FC, LLC, Wilmington DE 19801 (US)
(72) Inventor: SUN, Xuehui, Swedesboro, NJ New Jersey 08085 (US); NAPPA, Mario Joseph, Newark, DE Delaware 19711 (US)
(74) Representative: Dannenberger, Oliver Andre

(57) **Abstract**

The present disclosure relates to processes for reducing the concentration of R_{f}C=CX impurities in fluoroolefins. The process involves: contacting a mixture comprising at least one fluoroolefin and at least one R_{f}C=CX impurity with at least one amine to reduce the concentration of the at least one R_{f}C=CX impurity in the mixture; wherein R_{f} is a perfluorinated alkyl group, and X is H, F, Cl, Br or I. The present disclosure also relates to processes for making at least one hydrotetrafluoropropene product selected from the group consisting of CF₃CF=CH₂, CF₃CH=CHF, or at least one hydrochlorotrifluoropropene product selected from the group consisting of CF₃CC₁=CH₂, CF₃CH=CH₁, and mixtures thereof and reducing the concentration of CF₃C=CH impurity generated during the processes

## Description

### BACKGROUND

### Field of the Disclosure

The present disclosure relates to a process for reducing the concentration of R_{f}C≡CX impurities in fluoroolefins by contact with an amine.

### Description of Related Art

Many industries have been working for the past few decades to find replacements for the ozone depleting chlorofluorocarbons (CFCs) and hydrochlorofluorocarbons (HCFCs). The CFCs and HCFCs have been employed in a wide range of applications, including their use as aerosol propellants, refrigerants, cleaning agents, expansion agents for thermoplastic and thermoset foams, heat transfer media, gaseous dielectrics, fire extinguishing and suppression agents, power cycle working fluids, polymerization media, particulate removal fluids, carrier fluids, buffing abrasive agents, and displacement drying agents. In the search for replacements for these versatile compounds, many industries have turned to the use of hydrofluorocarbons (HFCs).

The HFCs do not contribute to the destruction of stratospheric ozone, but are of concern due to their contribution to the "greenhouse effect", i.e., they contribute to global warming. As a result of their contribution to global warming, the HFCs have come under scrutiny, and their widespread use may also be limited in the future. Thus, there is a need for chemical compounds that have both low ozone depleting potentials (ODPs) and low global warming potentials (GWPs).

### BRIEF SUMMARY OF THE DISCLOSURE

The present disclosure provides a process comprising: contacting a mixture comprising at least one fluoroolefin and at least one R_{f}C≡CX impurity with at least one amine to reduce the concentration of said at least one R_{f}C≡CX impurity in said mixture; wherein R_{f} is a perfluorinated alkyl group, and X is H, F, Cl, Br or I.

The present disclosure also provides a process for making at least one hydrotetrafluoropropene product selected from the group consisting of CF₃CF=CH₂, CF₃CH=CHF, and mixtures thereof. The process comprises: (a) dehydrohalogenating at least one starting material selected from the group consisting of CF₃CFClCH₃, CF₃CHFCH₂Cl, CF₃CHClCH₂F, CF₃CH₂CHFCl, CF₃CHFCH₂F, CF₃CH₂CF₂H, CF₃CF₂CH₃, and mixtures thereof to produce a product mixture comprising CF₃C≡CH impurity and said at least one hydrotetrafluoropropene product; (b) contacting said product mixture with at least one amine to reduce the concentration of said CF₃C≡CH impurity in said product mixture; and (c) recovering said at least one hydrotetrafluoropropene product having reduced concentration of said CF₃C≡CH impurity.

The present disclosure also provides a process for making at least one hydrochlorotrifluoropropene product selected from the group consisting of CF₃CCl=CH₂, CF₃CH=CHCl, and mixtures thereof. The process comprises: (a) dehydrohalogenating at least one starting material selected from the group consisting of CF₃CCl₂CH₃, CF₃CHClCH₂Cl, CF₃CHClCH₂F, CF₃CH₂CHCl₂, CF₃CHFCH₂Cl, CF₃CFClCH₃, CF₃CH₂CHFCl, and mixtures thereof to produce a product mixture comprising CF₃C≡CH impurity and said at least one hydrochlorotrifluoropropene product; (b) contacting said product mixture with at least one amine to reduce the concentration of said CF₃C≡CH impurity in said product mixture; and (c) recovering said at least one hydrochlorotrifluoropropene product having reduced concentration of said CF₃C≡CH impurity.

### DETAILED DESCRIPTION

Fluoroolefins have been found to have low ODPs and low GWPs and have been regarded as potential replacements for HFCs in many applications. For example, CF₃CF=CH₂ (HFO-1234yf) and CF₃CH=CHF (HFO-1234ze), having zero ODPs and low GWPs, have been identified as potential refrigerants. For another example, CF₃CH=CHCl (HCFO-1233zd) and CF₃CCl=CH₂ (HCFO-1233xf), having low ODPs and low GWPs, may be used as foam expansion agents. HCFO-1233zd is also an intermediate in the production of HFO-1234ze, and HCFO-1233xf is an intermediate in the production of HFO-1234yf.

It has been found that R_{f}C≡CX impurities, such as CF₃C≡CH, are often present in the fluoroolefin products. Since R_{f}C≡CX impurities might be highly toxic, they need to be removed from the fluoroolefin products.

The foregoing general description and the following detailed description are exemplary and explanatory only and are not restrictive of the invention, as defined in the appended claims. Other features and benefits of any one or more of the embodiments will be apparent from the following detailed description, and from the claims.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Unless otherwise defined, all technical and scientific terms used herein have the same meaning as commonly understood by one of ordinary skill in the art to which this invention belongs. In case of conflict, the present specification, including definitions, will control. Although methods and materials similar or equivalent to those described herein can be used in the practice or testing of embodiments of the present invention, suitable methods and materials are described below. In addition, the materials, methods, and examples are illustrative only and not intended to be limiting.

When an amount, concentration, or other value or parameter is given as either a range, preferred range or a list of upper preferable values and/or lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range.

Before addressing details of embodiments described below, some terms are defined or clarified.

HFO-1234ze may exist as one of two configurational isomers, E or Z. HFO-1234ze as used herein refers to the isomers, E-HFO-1234ze or Z-HFO-1234ze, as well as any combinations or mixtures of such isomers.

HCFO-1233zd also may exist as one of two configurational isomers, E or Z. HCFO-1233zd as used herein refers to the isomers, E- HCFO-1233zd or Z-HCFO-1233zd, as well as any combinations or mixtures of such isomers.

CF₃CF=CHCl (HCFO-1224yd) also may exist as one of two configurational isomers, *E* or *Z*. HCFO-1224yd as used herein refers to the isomers, *E*-HCFO-1224yd or Z-HCFO-1224yd, as well as any combinations or mixtures of such isomers.

CF₃CCl=CHCl (HCFO-1223xd) also may exist as one of two configurational isomers, *E* or *Z*. HCFO-1223xd as used herein refers to the isomers, *E*-HCFO-1223xd or *Z*-HCFO-1223xd, as well as any combinations or mixtures of such isomers.

The term "R_{f}C=CX impurity", as used herein, means the impurity of the formula R_{f}C≡CX present in a fluoroolefin product.

The term "fluoroolefin", as used herein, means a molecule containing hydrogen, carbon, optionally chlorine, fluorine, and a carbon-carbon double bond.

The term "hydrofluoroolefin", as used herein, means a molecule containing hydrogen, carbon, fluorine, and a carbon-carbon double bond.

The term "hydrochlorofluoroolefin", as used herein, means a molecule containing hydrogen, carbon, chlorine, fluorine, and a carbon-carbon double bond.

Unless indicated to the contrary, the term "alkyl", as used herein, either alone or in compound words, such as "perfluorinated alkyl group", includes cyclic or acyclic and straight-chain or branched alkyl groups having 1-20 carbon atoms. The term lower alkyl denotes an alkyl group having 1-6 carbon atoms. Examples of alkyl include groups, such as, methyl, ethyl, *n*-propyl, *i*-propyl, n-butyl, sec-butyl, tert-butyl, isobutyl, n-pentyl, isopentyl, neopentyl, 3-methylbutane, 2, 3-dimethyl-propane, n-hexyl, and the various other isomers of n-hexyl. In addition, the term includes saturated cycloalkyl groups containing 3-14 ring carbon atoms, such as cyclopropyl, cyclobutyl, cyclopentyl and cyclohexyl. It may be monocyclic or bicyclic.

The term "perfluorinated alkyl group", as used herein, means an alkyl group wherein all hydrogens on carbon atoms have been substituted by fluorines. In an embodiment, the perfluorinated alkyl groups have 1 to 3 carbon atoms. Examples of a perfluorinated alkyl group include -CF₃ and -CF₂CF₃

The term "alkoxy" refers to a group of the formula O-alkyl, wherein alkyl is defined hereinabove. Examples include methoxy, ethoxy, propoxy, i-propoxy, butoxy, pentoxy, and the like.

The term "amine", as used herein, means a chemical compound or a functional group that contains a basic nitrogen atom with a lone pair of electrons. The amine may be a primary amine, a secondary amine or tertiary amine or mixture thereof. Examples are described hereinbelow. However, it includes compounds of the formula R1R2R3N, wherein each R1, R2, R3 are independently hydrogen, alkyl, aralkyl, or heteroalkyl group, wherein at least one of R1, R2 and R3 is other than hydrogen and wherein each R1, R2 and R3 are independently unsubstituted or substituted with such groups as hydroxyl, halogen, alkoxy, or amino groups. The term amine includes RNH₂, R₂NH, and R₃N, as described herein. Further, as used herein, the term also includes hydrazines, such as R1NHNH₂, where R1 is as defined herein. In addition, the term amine includes heterocyclic amines, as defined herein. The term amine excludes ammonium salts, whether NH₄⁺, primary, secondary or tertiary ammonium salts.

The term "aralkyl", as used herein, means an alkyl group wherein one or more hydrogens on carbon atoms have been substituted by an aryl group. By "aryl", it is meant an aromatic ring containing 6, 10, 14, 18 or 22 ring carbon atoms and up to a total of 30 carbon atoms. Examples of an aralkyl group include C₆H₅CH₂-, phenethyl and the like.

The term "heteroalkyl", as used herein, means an alkyl group wherein one or more carbon atoms of the alkyl backbone have been substituted by a heteroatom, such as, O, S or N. Examples of heteroalkyl groups include CH₃CH₂CH₂NHCH₂CH₂-, CH₃CH₂CH₂OCH₂CH₂-,and the like.

As used herein, the term "halogen" denotes F, Cl, Br and I.

The term "aromatic", unless indicated to the contrary, is synonymous to the term "aryl" and are used interchangeably.

"Aliphatic", as used herein denotes non-aromatic hydrocarbyl group. It may be completely saturated or partially unsaturated. The hydrocarbyl group may contain double bonds (alkenes) or triples bonds (alkynes). The aliphatic group may be cyclic, containing at 1 or more rings in which the ring atoms are carbon atoms. Examples include the alkyl groups, as defined hereinabove.

"Hydrocarbyl" refers to an organic compound containing carbon and hydrogen atoms. As used herein, it contains at least one carbon atom. Alkyl groups are an example of hydrocarbyl.

The term "polyamine", as used herein, means an organic compound having two or more primary amino groups (-NH₂). For example, the two or more primary amino groups may be substituted on an alkyl group, an aryl group, an aralkyl or a heteroalkyl or a heterocyclic, as defined herein.

The term "heterocyclic compound", as used herein, shall denote any three to eight membered monocyclic, saturated, partially unsaturated partially aromatic ring or completely aromatic ring structure wherein at least one of the carbon atoms in the ring is replaced with a heteroatom selected from the group consisting of O, N and S, wherein the ring contains 2 to seven ring carbon atoms and 1-3 heteroatoms,; or a nine to ten membered saturated, partially unsaturated or partially or fully aromatic bicyclic ring system containing at least one heteroatom selected from the group consisting of O, N and S and up to a total of four heteroatoms, and the remaining ring atoms being carbon atoms (eight to nine ring carbon atoms). If the heteroocyclic ring contains at least two ring heteroatoms, the ring heteroatoms may be the same or different. Examples of suitable heterocyclic compounds include, but are not limited to, pyrrolidine, dioxalane, imidazolidine, pyrazoline, pyrazolidine, piperidine, dioxane, morpholine, thiomorpholine, piperazine, tetrahydrofuran, pyrroline (including 1-pyrroline, 2-pyrroline and 3-pyrroline), pyrazole, pyridine, pyrimidine, purine, pyridazine, bipyridine (including 2,2'-bipyridine, 4,4'-bipyridine, 2,3'-bipyridine, and 3,4'bipyridine), indoline, isoindoline, pyrazoline, imidazoline, pyrroline and the like.

The term "heterocyclic amine", as used herein, means a heterocyclic group wherein at least one member of its ring(s) is an amine nitrogen and at least two members of its ring(s) are carbon. As used herein, the heterocyclic amine includes heteroaromatic containing 6 to 10 ring atoms and 1, 2, or 3 nitrogen ring atoms and the remainder of the ring atoms being carbon atoms, such as pyrrole, pyridine, purine, pyrimidine, imidazole, pyrazole, pyrazine, pyridazine, indole, quinoline, isoquinoline, naphthyridine, quinoxaline, quinazoline and the like. In addition, the term includes heterocyclic amine containing 6-10 ring atoms having 1, 2 or 3 nitrogen atoms and 1 or 2 additional ring heteroatoms consisting of S or O, with the remaining ring carbon atoms being carbon atoms.

The term "aliphatic amine", as used herein, means an amine wherein the groups attached to nitrogen are aliphatic.

The term "ppm", as used herein, means parts per million by weight.

The term "ppm-molar", as used herein, means parts per million by mole.

The term "substantially free", as used herein, means that the fluoroolefin contains 2 ppm-molar or less of the R_{f}C≡CX impurity.

The term "dehydrohalogenation", as used herein, means dehydrofluorination or dehydrochlorination. The term "dehydrohalogenating", as used herein, means dehydrofluorinating or dehydrochlorinating. The term "dehydrohalogenated", as used herein, means dehydrofluorinated or dehydrochlorinated.

The term "dehydrofluorination", "dehydrofluorinating" or "dehydrofluorinated", as used herein, means a process during which hydrogen and fluorine on adjacent carbons in a molecule are removed.

The term "dehydrochlorination", "dehydrochlorinating", or "dehydrochlorinated", as used herein, means a process during which hydrogen and chlorine on adjacent carbons in a molecule are removed.

The term "phase transfer catalyst", as used herein, means a substance that facilitates the migration of a chemical compound from one phase into another phase. In some embodiments of this invention, the phase transfer catalyst is selected from the group consisting of crown ethers, onium salts, cryptands, polyalkylene glycols, and mixtures and derivatives thereof. The phase transfer catalyst can be ionic or neutral. In some embodiments of this invention, onium salts include quaternary phosphonium salts and quaternary ammonium salts. Examples of quaternary ammonium salts include tetra-n-butylammonium hydroxide, tetramethylammonium chloride, tetramethylammonium bromide, benzyltriethylammonium chloride, methyltri-n-octylammonium chloride (also known as Aliquat™ 336), dodecyltrimethylammonium bromide, tetra-n-butylammonium chloride, tetra-n-butylammonium bromide, tetra-n-butylammonium hydrogen sulfate, tetra-n-butylphosphonium chloride, tetraphenylphosphonium bromide, tetraphenylphosphonium chloride, triphenylmethylphosphonium bromide, triphenylmethylphosphonium chloride, and mixtures thereof. In this disclosure, a phase transfer catalyst might be used in the contacting step process to help removing the R_{f}C≡CX impurity from the fluoroolefin. A phase transfer catalyst might also be used in the dehydrohalogenation process with the basic aqueous solution.

The present disclosure provides a process for reducing the amount of R_{f}C≡CX impurity from fluoroolefin by contacting fluoroolefin containing R_{f}C≡CX impurity with amine. The process comprises: contacting a mixture comprising at least one fluoroolefin and at least one R_{f}C≡CX impurity with at least one amine to reduce the concentration of said at least one R_{f}C≡CX impurity in said mixture; wherein R_{f} is a perfluorinated alkyl group, and X is H, F, Cl, Br or I. In some embodiments of this invention, the process further comprises recovering said at least one fluoroolefin having reduced concentration of said at least one R_{f}C≡CX impurity.

In some embodiments of this invention, the amount of the at least one fluoroolefin in the mixture is at least 50 wt % based on the total weight of the mixture. In some embodiments of this invention, the amount of the at least one fluoroolefin in the mixture is at least 70 wt % based on the total weight of the mixture. In some embodiments of this invention, the amount of the at least one fluoroolefin in the mixture is at least 90 wt % based on the total weight of the mixture. In some embodiments of this invention, the mixture consists essentially of the at least one fluoroolefin and the at least one R_{f}C≡CX impurity.

A fluoroolefin in this disclosure can be a hydrofluoroolefin or a hydrochlorofluoroolefin. In some embodiments of this invention, the at least one fluoroolefin is hydrofluoroolefin. In some embodiments of this invention, the at least one fluoroolefin is hydrochlorofluoroolefin. In some embodiments of this invention, the at least one hydrofluoroolefin is selected from the group consisting of CF₃CF=CH₂ (HFO-1234yf), CF₃CH=CHF (HFO-1234ze), CF₃CH=CH₂ (HFO-1243zf), CF₃CH=CF₂ (HFO-1225zc), CF₃CF=CHF (HFO-1225ye), and mixtures thereof. In some embodiments of this invention, the at least one hydrochlorofluoroolefin is selected from the group consisting of CF₃CCl=CH₂ (HCFO-1233xf), CF₃CH=CHCl (HCFO-1233zd), CF₃CF=CHCl (HCFO-1224yd), CF₃CH=CCl₂ (HCFO-1223za), CF₃CCl=CHCl (HCFO-1223xd), CF₃CH=CFCl, CF₃CCl=CHF, and mixtures thereof. In some embodiments of this invention, the at least one fluoroolefin is selected from the group consisting of CF₃CF=CH₂, CF₃CH=CHF, CF₃CH=CH₂, CF₃CCl=CH₂, CF₃CH=CHCl, CF₃CH=CFCl, CF₃CH=CF₂, CF₃CCl=CHF, CF₃CF=CHF, CF₃CF=CHCl, CF₃CH=CCl₂, CF₃CCl=CHCl, and mixtures thereof. In some embodiments of this invention, the hydrofluoroolefin is at least one hydrotetrafluoropropene product selected from the group consisting of CF₃CF=CH₂, CF₃CH=CHF, and mixtures thereof. In some embodiments of this invention, the hydrochlorofluoroolefin is at least one hydrochlorotrifluoropropene product selected from the group consisting of CF₃CCl=CH₂, CF₃CH=CHCl, and mixtures thereof.

During the processes of making fluoroolefin and its precursors, R_{f}C≡CX impurities may be generated as byproducts. For example, during the dehydrochlorination process of CF₃CFClCH₃ (HCFC-244bb) to make HFO-1234yf, CF₃C≡CH impurity has been found present in the product mixture with HFO-1234yf. CF₃C≡CH impurity and/or CF₃C≡CCl impurity may also be present in the HCFC-244bb starting material.

The R_{f}C≡CX impurity that is removed from fluoroolefin by processes of this disclosure is a fluorinated terminal alkyne. In some embodiments of this invention, R_{f} is -CF₃. In some embodiments of this invention, R_{f} is -CF₂CF₃. In some embodiments of this invention, the at least one R_{f}C≡CX impurity is selected from the group consisting of CF₃C≡CH, CF₃C≡CCl, CF₃C≡CF, and mixtures thereof. In some embodiments of this invention, the at least one R_{f}C≡CX impurity is selected from the group consisting of CF₃C≡CH, CF₃C≡CCl, and mixtures thereof. In some embodiments of this invention, the at least one R_{f}C≡CX impurity is CF₃C≡CH. In some embodiments of this invention, the at least one R_{f}C≡CX impurity is CF₃C≡CCl.

In some embodiments of this invention, the at least one fluoroolefin is selected from the group consisting of CF₃CF=CH₂, CF₃CH=CHF, CF₃CH=CH₂, CF₃CCl=CH₂, CF₃CH=CHCl, CF₃CH=CFCl, CF₃CH=CF₂, CF₃CCl=CHF, CF₃CF=CHF, CF₃CF=CHCl, CF₃CH=CCl₂, CF₃CCl=CHCl, and mixtures thereof, and the at least one R_{f}C≡CX impurity is selected from the group consisting of CF₃C≡CH, CF₃C≡CCl, CF₃C≡CF, and mixtures thereof.

In some embodiments of this invention, the at least one fluoroolefin is selected from the group consisting of CF₃CF=CH₂, CF₃CH=CHF, CF₃CH=CH₂, CF₃CCl=CH₂, CF₃CH=CHCl, CF₃CF=CHCl, and mixtures thereof, and the at least one R_{f}C≡CX impurity is selected from the group consisting of CF₃C≡CH, CF₃C≡CCl, CF₃C≡CF, and mixtures thereof.

In some embodiments of this invention, the at least one fluoroolefin is CF₃CH=CH₂, and the at least one R_{f}C≡CX impurity is selected from the group consisting of CF₃C≡CH, CF₃C≡CCl, CF₃C≡CF, and mixtures thereof.

In some embodiments of this invention, the at least one fluoroolefin is selected from the group consisting of CF₃CF=CH₂, CF₃CH=CHF, CF₃CCl=CH₂, CF₃CH=CHCl, and mixtures thereof, and the at least one R_{f}C≡CX impurity is selected from the group consisting of CF₃C≡CH, CF₃C≡CCl, and mixtures thereof.

In some embodiments of this invention, the at least one fluoroolefin is selected from the group consisting of CF₃CF=CH₂, CF₃CH=CHF, CF₃CCl=CH₂, CF₃CH=CHCl, and mixtures thereof, and the at least one R_{f}C≡CX impurity is CF₃C≡CH.

In some embodiments of this invention, the at least one fluoroolefin is CF₃CF=CH₂, and the at least one R_{f}C≡CX impurity is selected from the group consisting of CF₃C≡CH, CF₃C≡CCl, and mixtures thereof.

In some embodiments of this invention, the at least one fluoroolefin is CF₃CF=CH₂, and the at least one R_{f}C≡CX impurity is CF₃C≡CH.

In some embodiments of this invention, the at least one fluoroolefin is CF₃CH=CHF, and the at least one R_{f}C≡CX impurity is selected from the group consisting of CF₃C≡CH, CF₃C≡CCl, and mixtures thereof.

In some embodiments of this invention, the at least one fluoroolefin is CF₃CH=CHF, and the at least one R_{f}C≡CX impurity is CF₃C≡CH.

In some embodiments of this invention, the at least one fluoroolefin is a mixture of CF₃CF=CH₂ and CF₃CH=CHF, and the at least one R_{f}C≡CX impurity is selected from the group consisting of CF₃C≡CH, CF₃C≡CCl, and mixtures thereof.

In some embodiments of this invention, the at least one fluoroolefin is a mixture of CF₃CF=CH₂ and CF₃CH=CHF, and the at least one R_{f}C≡CX impurity is CF₃C≡CH.

In some embodiments of this invention, the at least one fluoroolefin is selected from the group consisting of CF₃CCl=CH₂, CF₃CH=CHCl, and mixtures thereof, and the at least one R_{f}C≡CX impurity is selected from the group consisting of CF₃C≡CH, CF₃C≡CCl, and mixtures thereof.

In some embodiments of this invention, the at least one fluoroolefin is selected from the group consisting of CF₃CCl=CH₂, CF₃CH=CHCl, and mixtures thereof, and the at least one R_{f}C≡CX impurity is CF₃C≡CH.

In some embodiments of this invention, the at least one fluoroolefin is CF₃CH=CHCl, and the at least one R_{f}C≡CX impurity is selected from the group consisting of CF₃C≡CH, CF₃C≡CCl, and mixtures thereof.

In some embodiments of this invention, the at least one fluoroolefin is CF₃CH=CHCl, and the at least one R_{f}C≡CX impurity is CF₃C≡CH.

In some embodiments of this invention, the at least one fluoroolefin is CF₃CCl=CH₂, and the at least one R_{f}C≡CX impurity is selected from the group consisting of CF₃C≡CH, CF₃C≡CCl, and mixtures thereof.

In some embodiments of this invention, the at least one fluoroolefin is CF₃CCl=CH₂, and the at least one R_{f}C≡CX impurity is CF₃C≡CH.

It has been found through experiments that R_{f}C≡CX impurity can be removed from fluoroolefin by contacting with an amine.

In some embodiments of this invention, the at least one amine can be represented by the formula R₃N wherein each R is independently a hydrogen, an alkyl group, a heteroalkyl group, an aryl group, or an aralkyl group. The alkyl group, heteroalkyl group, aryl group, and aralkyl group can be substituted or unsubstituted. Substituted alkyl group, substituted heteroalkyl group, substituted aryl group, or substituted aralkyl group herein means that one or more hydrogens on carbon atoms have been substituted by functional groups, such as hydroxyl groups, alkoxy groups, halogens, amino groups, and the like. The at least one amine for this disclosure can be aliphatic amine, aromatic amine, or mixtures thereof. In some embodiments of this invention, the at least one amine is aliphatic amine.

In some embodiments of this invention, the at least one amine can be primary amine, secondary amine, tertiary amine, or mixtures thereof. In some embodiments of this invention, the at least one amine is primary unsubstituted alkyl amine of the formula RNH₂ wherein R is a C₁-C₁₆ unsubstituted alkyl group. In some embodiments of this invention, the at least one amine is primary unsubstituted alkyl amine of the formula RNH₂ wherein R is a C₄-C₁₂ unsubstituted alkyl group. Examples of primary unsubstituted alkyl amine include methylamine, ethylamine, propylamine, isopropylamine, butylamine, sec-butylamine, *tert*-butylamine, amylamine, isoamylamine, tert-amylamine, hexylamine, heptylamine, octylamine, *tert*-octylamine (1,1,3,3-tetramethylbutylamine), and mixtures thereof.

In some embodiments of this invention, the at least one amine is secondary unsubstituted alkyl amine of the formula R₂NH wherein each R is independently a C₁-C₁₄ unsubstituted alkyl group. In some embodiments of this invention, the at least one amine is secondary unsubstituted alkyl amine of the formula R₂NH wherein each R is independently a C₃-C₁₀ unsubstituted alkyl group. Examples of secondary unsubstituted alkyl amine include dimethylamine, diethylamine, dipropylamine, diisopropylamine, dibutylamine, di-sec-butylamine, diamylamine, dihexylamine, and mixtures thereof.

In some embodiments of this invention, the at least one amine is tertiary unsubstituted alkyl amine of the formula R₃N wherein each R is independently a C₁-C₁₂ unsubstituted alkyl group. In some embodiments of this invention, the at least one amine is tertiary unsubstituted alkyl amine of the formula R₃N wherein each R is independently a C₂-C₈ unsubstituted alkyl group. Examples of tertiary unsubstituted alkyl amine include trimethylamine, triethylamine, tripropylamine, tributylamine, triamylamine, trihexylamine, *N*,*N*-dimethylethylamine, *N*,*N*-dimethylpropylamine, *N,N-*dimethylbutylamine, and mixtures thereof.

In some embodiments of this invention, the at least one amine is selected from the group consisting of methylamine, dimethylamine, trimethylamine, ethylamine, diethylamine, triethylamine, propylamine, isopropylamine, dipropylamine, diisopropylamine, tripropylamine, butylamine, sec-butylamine, tert-butylamine, dibutylamine, tributylamine, di-*sec*-butylamine, amylamine, isoamylamine, *tert-*amylamine, diamylamine, triamylamine, hexylamine, dihexylamine, trihexylamine, heptylamine, octylamine, *tert*-octylamine (1,1,3,3-tetramethylbutylamine), *N,N-*dimethylethylamine, *N*,*N*-dimethylpropylamine, *N*,*N*-dimethylbutylamine, and mixtures thereof.

In some embodiments of this invention, at least one R group of the amine of the formula R₃N is a C₁-C₁₆ substituted alkyl group wherein one or more hydrogens on carbon atoms have been substituted by hydroxyl groups, and the rest of the R groups, if any, are independently selected from the group consisting of hydrogen and C₁-C₁₆ unsubstituted alkyl groups. Examples of such amine include ethanolamine (H₂NCH₂CH₂OH), diethanolamine, triethanolamine, tris(hydroxymethyl)aminomethane ((HOCH₂)₃CNH₂), 2-(methylamino)ethanol (CH₃NHCH₂CH₂OH), 2-(ethylamino)ethanol (CH₃CH₂NHCH₂CH₂OH), 2-(propylamino)ethanol (CH₃CH₂CH₂NHCH₂CH₂OH), 2-(isopropylamino)ethanol ((CH₃)₂CHNHCH₂CH₂OH), 2-(butylamino)ethanol (CH₃(CH₂)₃NHCH₂CH₂OH), 2-(*tert*-butylamino)ethanol ((CH₃)₃CNHCH₂CH₂OH), triisopropanolamine ([CH₃CH(OH)CH₂]₃N), *N,N-*dimethylethanolamine (HOCH₂CH₂N(CH₃)₂), 1-dimethylamino-2-propanol ((CH₃)₂NCH₂CH(OH)CH₃), 3-dimethylamino-1-propanol ((CH₃)₂N(CH₂)₃OH), 2-amino-2-methyl-1-propanol ((CH₃)₂C(NH₂)CH₂OH), and mixtures thereof.

In some embodiments of this invention, one R group of the amine of the formula R₃N is a C₁-C₁₆ substituted alkyl group wherein one or more hydrogens on carbon atoms have been substituted by hydroxyl groups, and the other two R groups are independently selected from the group consisting of hydrogen and C₁-C₁₆ unsubstituted alkyl groups. Examples of such amine include ethanolamine (H₂NCH₂CH₂OH), tris(hydroxymethyl)aminomethane ((HOCH₂)₃CNH₂), 2-(methylamino)ethanol (CH₃NHCH₂CH₂OH), 2-(ethylamino)ethanol (CH₃CH₂NHCH₂CH₂OH), 2-(propylamino)ethanol (CH₃CH₂CH₂NHCH₂CH₂OH), 2-(isopropylamino)ethanol ((CH₃)₂CHNHCH₂CH₂OH), 2-(butylamino)ethanol (CH₃(CH₂)₃NHCH₂CH₂OH), 2-(*tert-*butylamino)ethanol ((CH₃)₃CNHCH₂CH₂OH), *N*,*N*-dimethylethanolamine (HOCH₂CH₂N(CH₃)₂), 1-dimethylamino-2-propanol ((CH₃)₂NCH₂CH(OH)CH₃), 3-dimethylamino-1-propanol ((CH₃)₂N(CH₂)₃OH), 2-amino-2-methyl-1-propanol ((CH₃)₂C(NH₂)CH₂OH), and mixtures thereof.

In some embodiments of this invention, at least one R group of the amine of the formula R₃N is a C₁-C₁₆ substituted alkyl group wherein one or more hydrogens on carbon atoms have been substituted by amino groups, and the rest of the R groups, if any, are independently selected from the group consisting of hydrogen and C₁-C₁₆ unsubstituted alkyl groups. Examples of such amine include 3-(dimethylamino)propylamine ((CH₃)₂N(CH₂)₃NH₂), 3-(diethylamino)propylamine ((C₂H₅)₂N(CH₂)₃NH₂), and mixtures thereof.

In some embodiments of this invention, the at least one amine is polyamine. Examples of polyamine include ethylene diamine, 1,2-propylenediamine, 1,3-propylenediamine, 1,4-diaminobutane, 1,3-diaminopentane, 1,5-diaminopentane, 1,6-diaminohexane, 2-methyl-1,5-pentanediamine, spermidine (N-(3-aminopropyl)butane-1,4-diamine), spermine (*N*,*N*'-bis(3-aminopropyl)butane-1,4-diamine), diethylenetriamine, triethylenetetramine, and mixtures thereof.

In some embodiments of this invention, at least one R group of the amine of the formula R₃N is a C₂-C₁₆ substituted heteroalkyl group wherein one or more hydrogens on carbon atoms have been substituted by hydroxyl groups, and the rest of the R groups, if any, are independently selected from the group consisting of hydrogen and C₁-C₁₆ unsubstituted alkyl groups. In some embodiments of this invention, at least one R group of the amine of the formula R₃N is a C₂-C₁₆ substituted heteroalkyl group wherein the heteroatom of the heteroalkyl group is oxygen and wherein one or more hydrogens on carbon atoms have been substituted by hydroxyl groups, and the rest of the R groups, if any, are independently selected from the group consisting of hydrogen and C₁-C₁₆ unsubstituted alkyl groups. Examples of such amines include 2-[2-(dimethylamino)ethoxy]ethanol ((CH₃)₂NCH₂CH₂OCH₂CH₂OH), 2-(2-aminoethoxy)ethanol (H₂NCH₂CH₂OCH₂CH₂OH), and mixtures thereof.

In some embodiments of this invention, the at least one amine is heterocyclic amine. Examples of heterocyclic amine include pyrrolidine, pyrroline (including 1-pyrroline, 2-pyrroline and 3-pyrroline), piperidine, piperazine, morpholine, imidazole, pyrazole, pyridine, pyrimidine, pyridazine, pyrazine, pyridine, bipyridine (including 2,2'-bipyridine, 4,4'-bipyridine, 2,3'-bipyridine, and 3,4'-bipyridine, etc.), and mixtures thereof.

In some embodiments of this invention, the at least one amine is ammonia (NH₃). In some embodiments of this invention, the at least one amine is hydrazine (NH₂NH₂), hydrazine derivatives, such as alkyl hydrazines or arylhydrazines or aralkyl hydrazines and the like and mixtures thereof. Examples of hydrazine derivatives include methylhydrazine (CH₃NHNH₂), 1,1-dimethylhydrazine ((CH₃)₂NNH₂), 1,2-dimethylhydrazine (CH₃NHNHCH₃), phenylhydrazine, 2,4-dinitrophenylhydrazine, and mixtures thereof.

In some embodiments of this invention, the at least one amine is aromatic amine. Examples of aromatic amine include aniline, *o*-toluidine, *m*-toluidine, *p*-toluidine, xylidine, 2,4,6-trimethylaniline, *o*-anisidine, *m*-anisidine, *p*-anisidine, *N*-methylaniline, *N,N*-dimethylaniline, *N*-ethylaniline, *N*,*N*-diethylaniline, and mixtures thereof.

Mixtures of any of the aforementioned amines may also be used in this disclosure.

In some embodiments of this invention, the at least one amine is selected from the group consisting of amine of the formula R₃N, heterocyclic amines, hydrazine and its derivatives, and mixtures thereof, wherein each R is independently a hydrogen, an alkyl group, a heteroalkyl group, an aryl group, or an aralkyl group.

In some embodiments of this invention, the at least one amine is selected from the group consisting of amine of the formula R₃N, heterocyclic amines, and mixtures thereof, wherein each R is independently a hydrogen, an alkyl group, a heteroalkyl group, or an aralkyl group.

In the processes of removing the R_{f}C≡CX impurity from the fluoroolefin, sterically hindered amines, such as triphenylamine, 2,2,6,6-tetramethylpiperidine (TMP), and 2,2,6,6-tetramethyl-4-piperidinol might not be as effective as other amines. In some embodiments of this invention, the at least one amine does not include the sterically hindered amines. In some embodiments of this invention, the at least one amine does not include triphenylamine, 2,2,6,6-tetramethylpiperidine, and 2,2,6,6-tetramethyl-4-piperidinol.

In some embodiments of this invention, the at least one amine is in a solution with a suitable solvent during the contacting step. A suitable solvent in this disclosure means an inert solvent in which the at least one amine is at least partially soluble. The term "inert" herein means that the solvent shall not react with amine or fluoroolefin during the contacting step.

In some embodiments of this invention, the suitable solvent is selected from the group consisting of water, hydrocarbons, ethers, alcohols (including glycols), benzene and its derivatives, alkyl halides, alkyl nitriles, amides, sulfoxides, sulfones, phosphate esters, and mixtures thereof.

In some embodiments of this invention, the suitable solvent is selected from the group consisting of water, ethers, alcohols (including glycols), benzene and its derivatives, alkyl halides, alkyl nitriles, amides, sulfoxides, sulfones, and mixtures thereof.

Examples of ether include acyclic alkyl ethers, cyclic ethers, perfluorinated ethers, glyme, diglyme, triglyme, tetraglyme, and mixtures thereof. Examples of acyclic alkyl ether include dimethyl ether, diethyl ether, methyl ethyl ether, and mixtures thereof. Examples of cyclic ether include 2-methyltetrahydrofuran, tetrahydrofuran, tetrahydropyran, 1,4-dioxane, and mixtures thereof. Examples of perfluorinated ether include perfluoro-N-methyl morpholine, perfluorotetrahydrofuran, and mixtures thereof.

Examples of alcohol include alkyl alcohols, glycols, glycerol, and mixtures thereof. Examples of alkyl alcohol include methanol, ethanol, proponal, isopropanol, 2-methyl-2-propanol (*tert*-butanol), cyclohexanol, and mixtures thereof. Examples of glycol include ethylene glycol, propylene glycol, diethylene glycol, and mixtures thereof.

Examples of benzene and its derivatives include benzene, alkylbenzenes, halobenzenes, benzonitrile, phenol, anisole, biphenyl, nitrobenzene, and mixtures thereof. Examples of alkylbenzene include toluene, ethylbenzene, *o*-xylene, *m*-xylene, *p*-xylene, mesitylene, durene, 2-phenylhexane, and mixtures thereof. Examples of halobenzene include fluorobenzene, chlorobenzene, 1,2-dichlorobenzene, 1,4-dichlorobenzene, and mixtures thereof.

Examples of alkyl halide include dichloromethane, chloroform, carbon tetrachloride, chloroethane, 1,2-dichloroethane, and mixtures thereof.

Examples of alkyl nitrile include acetonitrile, propionitrile, butyronitrile, methyl glutaronitrile, adiponitrile, and mixtures thereof.

Examples of amide include N,N-dimethyl formamide, N,N-dimethyl acetamide, N-methyl-2-pyrrolidone, and mixtures thereof.

Examples of sulfoxide include dimethyl sulfoxide.

Examples of sulfone include sulfolane.

The contacting step of this disclosure can be carried out using well-known chemical engineering practices for scrubbing organic compounds, which includes continuous, semi-continuous or batch operations. In some embodiments of this invention, fluoroolefin containing R_{f}C≡CX impurity is mixed with amine, optionally in the presence of a suitable solvent, in a vessel equipped with an agitator. For example, fluoroolefin containing R_{f}C≡CX impurity may be contacted with amine, optionally in the presence of a suitable solvent, under a suitable amount of pressure to maintain liquid phase of the fluoroolefin and the amine in a vessel. The contents of the contacting vessel may be agitated to provide contact between the fluoroolefin and the amine. The fluoroolefin is then recovered by phase separation or distillation.

In some embodiments of this invention, the contacting step can be carried out by contacting a gaseous mixture of fluoroolefin and R_{f}C≡CX impurity with liquid amine (optionally in a solution with a suitable solvent). For example, the mixture comprising fluoroolefin and R_{f}C≡CX impurity may be bubbled into liquid amine (optionally in a solution with a suitable solvent) as a gas in a stirred vessel. The fluoroolefin is then allowed to leave the contacting vessel, optionally through a condenser, where it is collected for subsequent purification.

In some embodiments of this invention, the contacting step is conducted in a column packed with materials such as helices, rings, saddles, spheres or other formed shapes fabricated from glass, plastic, or ceramics. The mixture comprising fluoroolefin and R_{f}C≡CX impurity enters the bottom of the column as a vapor. The liquid amine (optionally in a solution with a suitable solvent) enters the top of the column, for example, by means of a pump connected to a reservoir of said liquid amine (optionally in a solution with a suitable solvent). The R_{f}C≡CX impurity in the fluoroolefin is then scrubbed off with the amine in the column and the fluoroolefin vapor, with reduced R_{f}C≡CX impurity, passes out the top of the column and is then collected. The amine passes out the bottom of the column and returns to the reservoir.

Optionally, a phase transfer catalyst can be employed in the contacting step process to increase the efficiency of removing the R_{f}C≡CX impurity from the fluoroolefin.

In some embodiments of this invention, the temperature during the contacting step is from about 0 °C to about 60 °C. In some embodiments of this invention, the temperature during the contacting step is from about 10 °C to about 30 °C. Typically, less reactive amines require relatively higher temperatures. The pressure during the contacting step is not critical and can be subatmospheric, atmospheric or superatmospheric. In some embodiments of this invention, the contacting step is carried out under superatmospheric pressure. In some embodiments of this invention, the contacting step is carried out under atmospheric pressure. The time of contact between the mixture comprising fluoroolefin and R_{f}C≡CX impurity and the amine is not critical and typically may be on the order of about 0.1 seconds to about an hour. In some embodiments of this invention, the contact time is from about 0.1 seconds to about 10 minutes.

During the contacting step, the mixture of fluoroolefin and R_{f}C≡CX impurity is scrubbed with amine in the contacting vessel, and the R_{f}C≡CX impurity is removed. In some embodiments of this invention, the concentration of the at least one R_{f}C≡CX impurity in the mixture is reduced to 200 ppm or less. In some embodiments of this invention, the concentration of the at least one R_{f}C≡CX impurity in the mixture is reduced to 100 ppm or less. In some embodiments of this invention, the concentration of the at least one R_{f}C≡CX impurity in the mixture is reduced to 50 ppm or less. In some embodiments of this invention, the concentration of the at least one R_{f}C≡CX impurity in the mixture is reduced to 10 ppm or less. In some embodiments of this invention, the concentration of the at least one R_{f}C≡CX impurity in the mixture is reduced to 2 ppm or less.

The fluoroolefin having reduced concentration of the R_{f}C≡CX impurity obtained from the contacting step can be recovered using techniques well-known in the art, such as condensation, distillation or phase separation. In some embodiments of this invention, the fluoroolefin obtained from the contacting step may be contaminated with amine and can be purified by scrubbing with water or weak acidic solution. The resulting fluoroolefin may be dried with a molecular sieve and further purified by distillation. In some embodiments of this invention, the fluoroolefin obtained from the contacting step is recovered by fractional distillation to separate from amine and/or other contaminants. The amine distillate may be recycled for use in the contacting step.

In some embodiments of this invention, the recovered fluoroolefin is substantially free of the R_{f}C≡CX impurity. In some embodiments of this invention, the recovered HFO-1234yf is substantially free of the R_{f}C≡CX impurity. In some embodiments of this invention, the recovered HFO-1234ze is substantially free of the R_{f}C≡CX impurity. In some embodiments of this invention, the recovered HFO-1243zf is substantially free of the R_{f}C≡CX impurity. In some embodiments of this invention, the recovered HCFO-1233xf is substantially free of the R_{f}C≡CX impurity. In some embodiments of this invention, the recovered HCFO-1233zd is substantially free of the R_{f}C≡CX impurity.

The present disclosure also provides a process for making at least one hydrotetrafluoropropene product selected from the group consisting of CF₃CF=CH₂, CF₃CH=CHF, and mixtures thereof. The process comprises: (a) dehydrohalogenating at least one starting material selected from the group consisting of CF₃CFClCH₃, CF₃CHFCH₂Cl, CF₃CHClCH₂F, CF₃CH₂CHFCl, CF₃CHFCH₂F, CF₃CH₂CF₂H, CF₃CF₂CH₃, and mixtures thereof to produce a product mixture comprising CF₃C≡CH impurity and said at least one hydrotetrafluoropropene product; (b) contacting said product mixture with at least one amine to reduce the concentration of said CF₃C≡CH impurity in said product mixture; and (c) recovering said at least one hydrotetrafluoropropene product having reduced concentration of said CF₃C≡CH impurity.

The present disclosure also provides a process for making at least one hydrochlorotrifluoropropene product selected from the group consisting of CF₃CCl=CH₂, CF₃CH=CHCl, and mixtures thereof. The process comprises: (a) dehydrohalogenating at least one starting material selected from the group consisting of CF₃CCl₂CH₃, CF₃CHClCH₂Cl, CF₃CHClCH₂F, CF₃CH₂CHCl₂, CF₃CHFCH₂Cl, CF₃CFClCH₃, CF₃CH₂CHFCl, and mixtures thereof to produce a product mixture comprising CF₃C≡CH impurity and said at least one hydrochlorotrifluoropropene product; (b) contacting said product mixture with at least one amine to reduce the concentration of said CF₃C≡CH impurity in said product mixture; and (c) recovering said at least one hydrochlorotrifluoropropene product having reduced concentration of said CF₃C≡CH impurity.

In some embodiments of this invention, the dehydrohalogenation process is carried out by pyrolyzing (thermally dehydrohalogenating) the starting material to produce the hydrotetrafluoropropene or hydrochlorotrifluoropropene product. The term "pyrolyzing" or "pyrolysis", as used herein, means chemical change produced by heating in the absence of catalyst. By absence of catalyst is meant that no material or treatment is added to the pyrolysis reactor that increases the reaction rate by reducing the activation energy of the pyrolysis process.

Suitable reactors for pyrolysis may be of any shape consistent with the process. In some embodiments of this invention, the reactor is a cylindrical tube, either straight or coiled. Heat is applied to the outside of the tube, with the chemical reaction taking place on the inside of the tube. Of note are pyrolysis reactors wherein the flow of gases through the reactor is partially obstructed to cause back-mixing, i.e. turbulence, and thereby promote mixing of gases and good heat transfer. This partial obstruction can be conveniently obtained by placing packing within the interior of the reactor, filling its cross-section or by using perforated baffles. The reactor packing can be particulate or fibrillar, has an open structure like that of Raschig Rings or other packings with a high free volume to avoid the accumulation of coke and to minimize pressure drop, and permits a generally free flow of gas. In some embodiments of this invention, the reactor packing is in cartridge disposition for ease of insertion and removal. In some embodiments of this invention, the pyrolysis reactor is substantially empty which means that the free volume of the reaction zone is at least about 80%, preferably at least about 90%, and more preferably at least about 95%. The free volume is the volume of the reaction zone minus the volume of the material that makes up the reactor packing. In some embodiments of this invention, the pyrolysis reactor is comprised of materials which are resistant to corrosion including stainless steel, Hastelloy™, Inconel™, Monel™, gold, or gold-lined or quartz.

The dehydrohalogenation process of this disclosure can be either a dehydrofluorination process or a dehydrochlorination process depending on the starting material and the corresponding fluoroolefin product. Typically, the pyrolysis temperature for dehydrofluorination is higher than the one for dehydrochlorination. In some embodiments of this invention, the dehydrofluorinating pyrolysis is conducted at a temperature of from about 600° C to about 900° C. In some embodiments of this invention, the dehydrochlorinating pyrolysis is conducted at a temperature of from about 400° C to about 700° C. Pyrolysis processes have also been disclosed in U.S. Patent No. 7,833,434, U.S. Patent Publication No. 2010-0105967, and U.S. Patent Publication No. 2010-0105967.

In some embodiments of this invention, the dehydrohalogenation process is carried out in the presence of a catalyst. Suitable catalysts for dehydrohalogenation include alumina, fluorided alumina, aluminum fluoride, aluminum chlorofluoride; metal compounds supported on alumina, fluorided alumina, aluminum fluoride, or aluminum chlorofluoride; chromium oxide (Cr₂O₃), fluorided chromium oxide, and cubic chromium trifluoride; oxides, fluorides, and oxyfluorides of magnesium, zinc and mixtures of magnesium and zinc and/or aluminum; lanthanum oxide and fluorided lanthanum oxide; carbon, and metal compounds supported on carbon. The metal compounds are oxides, fluorides, and oxyfluorides of at least one metal selected from the group consisting of sodium, potassium, rubidium, cesium, yttrium, lanthanum, cerium, praseodymium, neodymium, samarium, chromium, iron, cobalt, rhodium, nickel, copper, zinc, and mixtures thereof. In some embodiments of this invention, the dehydrohalogenation catalyst is selected from the group consisting of carbon, alumina, fluorided alumina, and mixtures thereof. In some embodiments of this invention, carbon includes acid-washed carbon, activated carbon and three dimensional matrix carbonaceous materials. In some embodiments of this invention, the dehydrohalogenation catalyst comprises alkali metal salt supported on chromium oxide. The catalytic dehydrohalogenation processes have also been disclosed in U.S. Patent No. 7,943,015, U.S. Patent No. 7,897,823, and U.S. Patent No. 7,985,884.

In some embodiments of this invention, the dehydrohalogenation process is carried out by reacting the starting material with a basic aqueous solution to produce the hydrotetrafluoropropene or hydrochlorotrifluoropropene product. As used herein, the basic aqueous solution is a liquid that is primarily an aqueous liquid having a pH of over 7, and the liquid may be a solution, dispersion, emulsion, suspension or the like. In some embodiments of this invention, the basic aqueous solution has a pH of 8 or higher. In some embodiments of this invention, the basic aqueous solution has a pH of 10 or higher. Typically, a dehydrofluorination process needs a higher pH solution than a dehydrochlorination process.

In some embodiments of this invention, an inorganic base is used to form the basic aqueous solution. Such inorganic base can be selected from the group consisting of hydroxide, oxide, carbonate, and phosphate salts of alkali, alkaline earth metals and mixtures thereof. In some embodiments, such inorganic base is sodium hydroxide, potassium hydroxide, or mixtures thereof. In some embodiments of this invention, the basic aqueous solution is an aqueous solution of a quaternary ammonium hydroxide of the formula NR'₄OH wherein each R' is independently hydrogen, a C₁ to C₁₆ alkyl group, aralkyl group, or substituted alkyl group, provided that not all R' are hydrogens. Examples of NR'₄OH compound include tetra-n-butylammonium hydroxide, tetra-n-propylammonium hydroxide, tetraethylammonium hydroxide, tetramethylammonium hydroxide, benzyltrimethylammonium hydroxide, hexadecyltrimethyammonium hydroxide, choline hydroxide, and mixtures thereof.

Optionally, the starting material is reacted with the basic aqueous solution in the presence of an organic solvent. In some embodiments of this invention, the organic solvent is selected from the group consisting of benzene and its derivatives, alcohols, alkyl halides, alkyl nitriles, ethers, amides, ketones, sulfoxides, phosphate esters and mixtures thereof.

Optionally, the starting material is reacted with the basic aqueous solution in the presence of a phase transfer catalyst. The phase transfer catalyst used herein can facilitate the transfer of ionic compounds into an organic phase from an aqueous phase and facilitate the reaction between water-soluble and water-insoluble reaction components.

The dehydrohalogenation using a basic aqueous solution has also been disclosed in PCT Publication No. WO2010/129844.

In some embodiments of this invention, during the dehydrohalogenating step, starting materials selected from the group consisting of CF₃CFClCH₃ (HCFC-244bb), CF₃CHFCH₂Cl (HCFC-244eb), CF₃CHClCH₂F (HCFC-244db), CF₃CH₂CHFCl (HCFC-244fa), CF₃CHFCH₂F (HFC-245eb), CF₃CH₂CF₂H (HFC-245fa), and CF₃CF₂CH₃ (HFC-245cb) are dehydrohalogenated to form either CF₃CF=CH₂ or CF₃CH=CHF product. In some embodiments of this invention, the at least one starting material is selected from the group consisting of CF₃CFClCH₃, CF₃CHFCH₂Cl, CF₃CHFCH₂F, CF₃CF₂CH₃, and mixtures thereof, and the at least one hydrotetrafluoropropene product is CF₃CF=CH₂. In some embodiments of this invention, the at least one starting material is CF₃CFClCH₃, and the at least one hydrotetrafluoropropene product is CF₃CF=CH₂ (i.e., the starting material CF₃CFClCH₃ is dehydrochlorinated to produce a product mixture comprising CF₃CF=CH₂ product and CF₃C≡CH impurity). In some embodiments of this invention, the at least one starting material is CF₃CHFCH₂F, and the at least one hydrotetrafluoropropene product is CF₃CF=CH₂ (i.e., the starting material CF₃CHFCH₂F is dehydrofluorinated to produce a product mixture comprising CF₃CF=CH₂ product and CF₃C≡CH impurity). In some embodiments of this invention, the at least one starting material is selected from the group consisting of CF₃CH₂CHF₂, CF₃CH₂CHFCl, CF₃CHClCH₂F, and mixtures thereof, and the at least one hydrotetrafluoropropene product is CF₃CH=CHF.

In some embodiments of this invention, during the dehydrohalogenating step, starting materials selected from the group consisting of CF₃CCl₂CH₃ (HCFC-243ab), CF₃CHClCH₂Cl (HCFC-243db), CF₃CHClCH₂F (HCFC-244db), CF₃CH₂CHCl₂ (HCFC-243fa), CF₃CHFCH₂Cl (HCFC-244eb), CF₃CFClCH₃ (HCFC-244bb), and CF₃CH₂CHFCl (HCFC-244fa) are dehydrohalogenated to form either CF₃CCl=CH₂ or CF₃CH=CHCl product. In some embodiments of this invention, the at least one starting material is selected from the group consisting of CF₃CHClCH₂Cl, CF₃CH₂CHCl₂, and mixtures thereof, and the at least one hydrochlorotrifluoropropene product is CF₃CH=CHCl (i.e., the at least one starting material selected from the group consisting of CF₃CHClCH₂Cl, CF₃CH₂CHCl₂, and mixtures thereof is dehydrochlorinated to produce a product mixture comprising CF₃CH=CHCl product and CF₃C≡CH impurity). In some embodiments of this invention, the at least one starting material is selected from the group consisting of CF₃CHClCH₂Cl, CF₃CCl₂CH₃, and mixtures thereof, and the at least one hydrochlorotrifluoropropene product is CF₃CHCl=CH₂ (i.e., the at least one starting material selected from the group consisting of CF₃CHClCH₂Cl, CF₃CCl₂CH₃, and mixtures thereof is dehydrochlorinated to produce a product mixture comprising CF₃CHCl=CH₂ product and CF₃C≡CH impurity).

During the dehydrohalogenating step, byproduct CF₃C≡CH is also generated. During the contacting step, the product mixture of hydrotetrafluoropropene or hydrochlorotrifluoropropene product and CF₃C≡CH impurity is scrubbed with amine using the contacting step processes as described in this disclosure. In some embodiments of this invention, the at least one amine employed in the contacting step (b) to remove the CF₃C≡CH impurity from the hydrotetrafluoropropene or hydrochlorotrifluoropropene product mixture is selected from the group consisting of amine of the formula R₃N, heterocyclic amines, hydrazine and its derivatives, and mixtures thereof, wherein each R is independently a hydrogen, an alkyl group, a heteroalkyl group, an aryl group, or an aralkyl group. In some embodiments of this invention, the at least one amine employed in the contacting step (b) is selected from the group consisting of amine of the formula R₃N, heterocyclic amines, and mixtures thereof, wherein each R is independently a hydrogen, an alkyl group, a heteroalkyl group, or an aralkyl group.

In some embodiments of this invention, the concentration of the CF₃C≡CH impurity in the hydrotetrafluoropropene or hydrochlorotrifluoropropene product mixture is reduced to 200 ppm or less in the contacting step (b). In some embodiments of this invention, the concentration of the CF₃C≡CH impurity in the hydrotetrafluoropropene or hydrochlorotrifluoropropene product mixture is reduced to 100 ppm or less in the contacting step (b). In some embodiments of this invention, the concentration of the CF₃C≡CH impurity in the hydrotetrafluoropropene or hydrochlorotrifluoropropene product mixture is reduced to 50 ppm or less in the contacting step (b). In some embodiments of this invention, the concentration of the CF₃C≡CH impurity in the hydrotetrafluoropropene or hydrochlorotrifluoropropene product mixture is reduced to 10 ppm or less in the contacting step (b). In some embodiments of this invention, the concentration of the CF₃C≡CH impurity in the hydrotetrafluoropropene or hydrochlorotrifluoropropene product mixture is reduced to 2 ppm or less in the contacting step (b).

In some embodiments of this invention, the concentration of the CF₃C≡CH impurity in the product mixture comprising CF₃C≡CH impurity and CF₃CF=CH₂ product is reduced to 200 ppm or less in the contacting step (b). In some embodiments of this invention, the concentration of the CF₃C≡CH impurity in the product mixture comprising CF₃C≡CH impurity and CF₃CF=CH₂ product is reduced to 100 ppm or less in the contacting step (b). In some embodiments of this invention, the concentration of the CF₃C≡CH impurity in the product mixture comprising CF₃C≡CH impurity and CF₃CF=CH₂ product is reduced to 50 ppm or less in the contacting step (b). In some embodiments of this invention, the concentration of the CF₃C≡CH impurity in the product mixture comprising CF₃C≡CH impurity and CF₃CF=CH₂ product is reduced to 10 ppm or less in the contacting step (b). In some embodiments of this invention, the concentration of the CF₃C≡CH impurity in the product mixture comprising CF₃C≡CH impurity and CF₃CF=CH₂ product is reduced to 2 ppm or less in the contacting step (b).

In some embodiments of this invention, the concentration of the CF₃C≡CH impurity in the product mixture comprising CF₃C≡CH impurity and CF₃CH=CHF product is reduced to 200 ppm or less in the contacting step (b). In some embodiments of this invention, the concentration of the CF₃C≡CH impurity in the product mixture comprising CF₃C≡CH impurity and CF₃CH=CHF product is reduced to 100 ppm or less in the contacting step (b). In some embodiments of this invention, the concentration of the CF₃C≡CH impurity in the product mixture comprising CF₃C≡CH impurity and CF₃CH=CHF product is reduced to 50 ppm or less in the contacting step (b). In some embodiments of this invention, the concentration of the CF₃C≡CH impurity in the product mixture comprising CF₃C≡CH impurity and CF₃CH=CHF product is reduced to 10 ppm or less in the contacting step (b). In some embodiments of this invention, the concentration of the CF₃C≡CH impurity in the product mixture comprising CF₃C≡CH impurity and CF₃CH=CHF product is reduced to 2 ppm or less in the contacting step (b).

The hydrotetrafluoropropene or hydrochlorotrifluoropropene product obtained from the contacting step (b) can be recovered using the recovering step processes as described in this disclosure. In some embodiments of this invention, various azeotropic or azeotrope-like (i.e., near azeotrope) compositions of the hydrotetrafluoropropene or hydrochlorotrifluoropropene product may be utilized in the processes of recovering these products. For example, HF can be added to the HFO-1234yf product mixture obtained from the contacting step (b), and separation of HFO-1234yf includes isolation of azeotrope or near azeotrope of HFO-1234yf and HF and further processing to produce HF-free HFO-1234yf by using procedures similar to that disclosed in U.S. Patent No. 7,897,823. Azeotrope or near azeotrope compositions of HFO-1234yf and HF have been disclosed in U.S. Patent No. 7,476,771. For another example, HF can be added to the HFO-1234ze product mixture obtained from the contacting step (b), and separation of HFO-1234ze includes isolation of azeotrope or near azeotrope of HFO-1234ze and HF and further processing to produce HF-free HFO-1234ze by using procedures similar to that disclosed in U.S. Patent No. 7,897,823. U.S. Patent No. 7,423,188 discloses azeotrope or near-azeotrope compositions of the E- isomer of HFO-1234ze and HF, and U.S. Patent Publication No. 2010-0200798 discloses azeotrope or near-azeotrope compositions of the Z- isomer of HFO-1234ze and HF. For another example, HF can be added to the HCFO-1233xf product mixture obtained from the contacting step (b), and separation of HCFO-1233xf includes isolation of azeotrope or near azeotrope of HCFO-1233xf and HF and further processing to produce HF-free HCFO-1233xf by using procedures similar to that disclosed in U.S. Patent No. 7,897,823. The azeotrope compositions of HCFO-1233xf and HF has been disclosed in U.S. Patent Publication No. 2010-0072415. For another example, HF can be added to the HCFO-1233zd product mixture obtained from the contacting step (b), and separation of HCFO-1233zd includes isolation of azeotrope or near azeotrope of HCFO-1233zd and HF and further processing to produce HF-free HCFO-1233zd by using procedures similar to that disclosed in U.S. Patent No. 7,897,823. Some azeotrope compositions of HCFO-1233zd and HF have been disclosed in U.S. Patent No. 6,013,846.

In some embodiments of this invention, the recovered hydrotetrafluoropropene or hydrochlorotrifluoropropene product is substantially free of the CF₃C≡CH impurity. In some embodiments of this invention, the recovered HFO-1234yf product is substantially free of the CF₃C≡CH impurity. In some embodiments of this invention, the recovered HFO-1234ze product is substantially free of the CF₃C≡CH impurity.

The contacting vessels, reactors, distillation columns, and their associated feed lines, effluent lines, and associated units used in applying the processes of embodiments of this invention should be constructed of materials resistant to corrosion. Typical materials of construction include stainless steels, in particular of the austenitic type, the well-known high nickel alloys, such as Monel™ nickel-copper alloys, Hastelloy™ nickel-based alloys and, Inconel™ nickel-chromium alloys, and copper-clad steel.

of NbCl₅, a fluorinated species of MoCl₆, a fluorinated species of FeCl₃, or combinations thereof. Antimony pentachloride is most preferred.

These catalysts can be readily regenerated by any means known in the art if they become deactivated. One suitable method of regenerating the catalyst involves flowing a stream of chlorine through the catalyst. For example, from about 0.002 to about 0.2 lb per hour of chlorine can be added to the liquid phase reaction for every pound of liquid phase fluorination catalyst. This may be done, for example, for from about 1 to about 2 hours or continuously at a temperature of from about 65°C to about 100°C.

This second step of the reaction is not necessarily limited to a liquid phase reaction and may also be performed using a vapor phase reaction or a combination of liquid and vapor phases, such as that disclosed in U.S. Published Patent Application No. 20070197842, the contents of which are incorporated herein by reference. To this end, the HCFO-1233xf containing feed stream is preheated to a temperature of from about 50°C to about 400°C, and is contacted with a catalyst and fluorinating agent. Catalysts may include standard vapor phase agents used for such a reaction

and fluorinating agents may include those generally known in the art, such as, but not limited to, hydrogen fluoride.

In the third step of HFO-1234yf production, HCFC-244bb is fed to a second vapor phase reactor (dehydrochlorination reactor) to be dehydrochlorinated to make the desired product 2,3,3,3-tetrafluoropropene (HFO-1234yf). This reactor can either be non-catalytic or it can contain a catalyst that can catalytically dehydrochlorinate HCFC-244bb to make HFO-1234yf.

The catalysts, if present, may be metal halides, halogenated metal oxides, neutral (or zero oxidation state) metal or metal alloy, or activated carbon in bulk or supported form. Metal halide or metal oxide catalysts may include, but are not limited to, mono-, bi-, and tri-valent metal halides, oxides and their mixtures/combinations, and more preferably mono-, and bi-valent metal halides and their mixtures/combinations. Component metals include, but are not limited to, Cr³⁺, Fe³⁺, Mg²⁺, Ca²⁺, Ni²⁺, Zn²⁺, Pd²⁺, Li⁺, Na⁺, K⁺, and Cs⁺. Component halogens include, but are not limited to, F⁻, Cl⁻, Br⁻, and I⁻. Examples of useful mono- or bi-valent metal halide include, but are not limited to, LiF, NaF, KF, CsF, MgF₂, CaF₂, LiCl, NaCl, KCl, and CsCl. Halogenation treatments can include any of those known in the prior art, particularly those that employ HF, F₂, HCl, Cl₂, HBr, Br₂, HI, and I₂ as the halogenation source.

When neutral, i.e., zero valent, metals, metal alloys and their mixtures are used. Useful metals include, but are not limited to, Pd, Pt, Rh, Fe, Co, Ni, Cu, Mo, Cr, Mn, and combinations of the foregoing as alloys or mixtures. The catalyst may be supported or unsupported. Useful examples of metal alloys include, but are not limited to, SS 316, Monel 400, Incoloy 825, Alloy 20, Hastelloy, Inconel 600, and Inconel 625.

In an aspect of the present invention, in this step, catalysts include activated carbon, stainless steel (e.g., SS 316), austenitic nickel-based alloys (e.g., Inconel 625), nickel, and in certain embodiments fluorinated 10% CsCl/MgO. A suitable reaction temperature is about 300-550°C and a suitable reaction pressure may be between about 0-150 psig. The reactor effluent may be fed to a caustic scrubber or to a distillation column to remove the byproduct of HCl to produce an acid-free

### EXAMPLE OF USE OF PRESENT INVENTION

The removal of alkyne impurities such as CF₃C≡CH impurity from the final product, namely HFO-1234yf using techniques known in the art heretofore, will incur extra cost and possibly yield loss as well. For example, using distillation to bring down CF₃C≡CH impurity level from 50 - 100 ppm to 5 -12 ppm caused an HFO-1234yf loss of almost 10%. But, the present invention provides methods of removing HCFO-alkyne impurities, such as CF₃CECH, from the reactor feed to improve the overall efficiency of the HFO-1234yf conversion process.

An aspect of the present invention is the preparation of HFO-1234yf that is substantially free of 3,3,3-trifluoropropyne comprising:
(i) providing a starting composition including a compound of Formulae I, II, or III:

   CX₂=CCl-CH₂X (I);

   CX₃-CCl=CH₂ (II);

   or

   CX₃-CHCl-CH₂X (III)

   wherein X is independently selected from F, Cl, Br, and I, provided that at least one X is not fluorine;
(ii) contacting the starting composition with a first fluorinating agent to produce a first intermediate composition including 2-chloro-3,3,3-trifluoropropene and a first chlorine-containing byproduct;
(iii)contacting the first intermediate composition with a second fluorinating agent to produce a second intermediate composition including 2-chloro-1,1,1,2-tetrafluoropropane and 2-chloro-3,3,3-trifluoropropene;
(iv)dehydrochlorinating at least a portion of the second intermediate composition including 2-chloro-1,1,1,2-tetrafluoropropane and 2-chloro-3,3,3-trifluoropropene to produce a reaction product including 2,3,3,3-tetrafluoropropene and CF₃C≡CH impurity;
(v) contacting the reaction product of step iv with at least one amine and
(vi) recovering said 2,3,3,3-tetrafluoropropene produced in step (v) having reduced concentration of said CF₃C≡CH impurity.

In certain aspects, the preparation of HFO-1234yf generally includes at least three reaction steps, as follows:
(1) (CX₂=CCl-CH₂X or CX₃-CCl=CH₂ or CX₃-CHCl-CH₂X) + HF → 2-chloro-3,3,3-trifluoropropene (HCFO-1233xf) + HCl in a vapor phase reactor charged with a solid catalyst;
(2) 2-chloro-3,3,3-trifluoropropene (HCFO-1233xf) + HF → 2-chloro-1,1,1,2-tetrafluoropropane (HCFC-244bb) in a liquid phase reactor charged with a liquid hydrofluorination catalyst; and
(3) 2-chloro-1,1,1,2-tetrafluoropropane (HCFC-244bb) → 2,3,3,3-tetrafluoropropene (HFO-1234yf) in a vapor phase reactor.
wherein X is independently selected from F, Cl, Br, and I, provided that at least one X is not fluorine.

Generally speaking, the starting material of the first reaction step may be represented by one or more chlorinated compounds according to Formulas I, II, and/or III:

CX₂=CCl-CH₂X (Formula I)

CX₃-CCl=CH₂ (Formula (II)

CX₃-CHCl-CH₂X (Formula III)

wherein X is independently selected from F, Cl, Br, and I, provided that at least one X is not fluorine. In certain embodiments, these compounds contain at least one chlorine, a majority of X is chlorine, or all X is chlorine.

In the first step, such starting materials (which, in certain embodiments includes 1,1,2,3-tetrachloropropene (1230xa) and/or 1,1,1,2,3-pentachloropropane (HCC-240db)) is reacted with anhydrous HF in a first vapor phase reactor (fluorination reactor) to produce a mixture of at least HCFO-1233xf (2-chloro-3,3,3-trifluoropropene) and HCl. The reaction can be carried out at a temperature of about 200-400°C and a pressure of about 0-200 psig. The effluent stream exiting the vapor phase reactor may optionally comprise additional components, such as un-reacted HF, heavy intermediates, HCFC-244bb, HFC-245cb (1,1,1,2, 2-pentafluoropropane), or the like.

This reaction may be conducted in any reactor suitable for a vapor phase fluorination reaction. The reactor may be constructed from materials which are resistant to the corrosive effects of hydrogen fluoride and catalyst such as Hastalloy, Inconel, Monel. In case of a vapor phase process, the reactor is filled with a vapor phase fluorination catalyst. Any fluorination catalysts known in the art may be used in this process. Suitable catalysts include, but are not limited to chromium, aluminum, cobalt, manganese, nickel and iron oxides, hydroxides, halides, oxyhalides, inorganic salts thereof and their mixtures any of which may be optionally halogenated. Catalysts suitable for the present invention nonexclusively include Cr₂O₃, FeCl₃/C, Cr₂O₃/Al₂O₃, Cr₂O₃/AlF₃, Cr₂O₃/carbon, CoCl₂/Cr₂O₃/Al₂O₃, NiCl₂/Cr₂O₃/Al₂O₃, CoCl₂/AlF₃, NiCl₂/AlF₃ and mixtures thereof. Chromium oxide/aluminum oxide catalysts are described in U.S. Pat. No. 5,155,082, the contents of which are incorporated herein by reference. Chromium (III) oxides such as crystalline chromium oxide or amorphous chromium oxide are suitable catalysts and in an embodiment, the catalyst used in this step is amorphous chromium oxide. Chromium oxide (Cr₂O₃) is a commercially available material which may be purchased in a variety of particle sizes. Fluorination catalysts having a purity of at least 98% are preferred. The fluorination catalyst is present in an excess but in at least an amount sufficient to drive the reaction.

This first step of the reaction is not necessarily limited to a vapor phase reaction, as described above, but may also be performed using a liquid phase reaction or a combination of liquid and vapor phases, such as that disclosed in U.S. Published Patent Application No. 20070197842, the contents of which are incorporated herein by reference. It is also contemplated that the reaction can be carried out batch wise, continuously, or a combination of these. For embodiments in which the reaction comprises a liquid phase reaction, the reaction can be catalytic or non-catalytic. Lewis acid catalysts, such as metal-halide catalysts, including antimony halides, tin halides, thallium halides, iron halides, and combinations of two or more of these, may be employed. In certain embodiments, metal chlorides and metal fluorides are employed, including, but not limited to, SbCl₅, SbCl₃, SbF₅, SnCl₄, TiCl₄, FeCl₃ and combinations of two or more of these.

The effluent from the reactor may be optionally processed to achieve desired degrees of separation and/or other processing. By way of non-limiting example, the product effluent may contain one or more impurities, such as, HCl, unconverted reactants, and/or other by-products. These products may be removed using standard methods known or otherwise discussed herein. HCl, for example, can be recovered by conventional distillation, or using water or caustic scrubbers, as discussed in greater detail below, and the unreacted starting reagents isolated and recycled.

In the second step of the process for forming 2,3,3,3-tetrafluoropropene, HCFO-1233xf is converted to HCFC-244bb. In one embodiment, this step may be performed in the liquid phase in a liquid phase reactor, which may be PTFE or PFA-lined. Such a process may be performed in a temperature range of about 70-120°C and about 50-120 psig.

Any liquid phase fluorination catalyst may be used in the invention. A non-exhaustive list includes Lewis acids, transition metal halides, transition metal oxides, Group IVb metal halides, a Group Vb metal halides, or combinations thereof. Non-exclusive examples of liquid phase fluorination catalysts are an antimony halide, a tin halide, a tantalum halide, a titanium halide, a niobium halide, and molybdenum halide, an iron halide, a fluorinated chrome halide, a fluorinated chrome oxide or combinations thereof. Specific non-exclusive examples of liquid phase fluorination catalysts are SbCl₅, SbCl₃, SbF₅, SnCl₄, TaCl₅, TiCl₄, NbCl₅, MoCl₆, FeCl₃, a fluorinated species of SbCl₅, a fluorinated species of SbCl₃, a fluorinated species of SnCl₄, a fluorinated species of TaCl₅, a fluorinated species of TiCl₄, a fluorinated species of NbCl₅, a fluorinated species of MoCl₆, a fluorinated species of FeCl₃, or combinations thereof. Antimony pentachloride is most preferred.

These catalysts can be readily regenerated by any means known in the art if they become deactivated. One suitable method of regenerating the catalyst involves flowing a stream of chlorine through the catalyst. For example, from about 0.002 to about 0.2 lb per hour of chlorine can be added to the liquid phase reaction for every pound of liquid phase fluorination catalyst. This may be done, for example, for from about 1 to about 2 hours or continuously at a temperature of from about 65°C to about 100°C.

This second step of the reaction is not necessarily limited to a liquid phase reaction and may also be performed using a vapor phase reaction or a combination of liquid and vapor phases, such as that disclosed in U.S. Published Patent Application No. 20070197842, the contents of which are incorporated herein by reference. To this end, the HCFO-1233xf containing feed stream is preheated to a temperature of from about 50°C to about 400°C, and is contacted with a catalyst and fluorinating agent. Catalysts may include standard vapor phase agents used for such a reaction and fluorinating agents may include those generally known in the art, such as, but not limited to, hydrogen fluoride.

In the third step of HFO-1234yf production, HCFC-244bb is fed to a second vapor phase reactor (dehydrochlorination reactor) to be dehydrochlorinated to make the desired product 2,3,3,3-tetrafluoropropene (HFO-1234yf). This process is conducted as described herein, For example, in an embodiment, this reaction can either be non-catalytic or it can contain a catalyst that can catalytically dehydrochlorinate HCFC-244bb to make HFO-1234yf.

The catalysts, if present, may be metal halides, halogenated metal oxides, neutral (or zero oxidation state) metal or metal alloy, or activated carbon in bulk or supported form. Metal halide or metal oxide catalysts may include, but are not limited to, mono-, bi-, and tri-valent metal halides, oxides and their mixtures/combinations, and more preferably mono-, and bi-valent metal halides and their mixtures/combinations. Component metals include, but are not limited to, Cr³⁺, Fe³⁺, Mg²⁺, Ca²+, Ni²⁺, Zn²⁺, Pd²⁺, Li⁺, Na⁺, K⁺, and Cs⁺. Component halogens include, but are not limited to, F⁻, Cl⁻, Br⁻, and I⁻. Examples of useful mono- or bi-valent metal halide include, but are not limited to, LiF, NaF, KF, CsF, MgF₂, CaF₂, LiCl, NaCl, KCl, and CsCl. Halogenation treatments can include any of those known in the prior art, particularly those that employ HF, F₂, HCl, Cl₂, HBr, Br₂, HI, and I₂ as the halogenation source.

When neutral, i.e., zero valent, metals, metal alloys and their mixtures are used. Useful metals include, but are not limited to, Pd, Pt, Rh, Fe, Co, Ni, Cu, Mo, Cr, Mn, and combinations of the foregoing as alloys or mixtures. The catalyst may be supported or unsupported. Useful examples of metal alloys include, but are not limited to, SS 316, Monel 400, Incoloy 825, Alloy 20, Hastelloy, Inconel 600, and Inconel 625.

In an aspect of the present invention, catalysts include activated carbon, stainless steel (e.g., SS 316), austenitic nickel-based alloys (e.g., Inconel 625), nickel, and in certain embodiments fluorinated 10% CsCl/MgO. A suitable reaction temperature is about 300-550°C and a suitable reaction pressure may be between about 0-150 psig. The reactor effluent may be fed to a caustic scrubber or to a distillation column to remove the byproduct of HCl to produce an acid-free organic product which, optionally, may undergo further purification using one or any combination of purification techniques that are known in the art.

The reaction may be carried out at a temperature range of from about 200°C to about 800° C, from about 300°C to about 600°C, or from about 400°C to about 500°C. Suitable reactor pressures range from about 0 psig to about 200 psig, from about 10 psig to about 100 psig, or from about 20 to about 70 psig.

In general, the effluent from the dehydrochlorination reactor may be processed to achieve desired degrees of separation and/or other processing. Besides HFO-1234yf produced, the effluent generally contains HCl, unconverted HCFC-244bb, and HCFO-1233xf (which is mainly carried over from the previous step of HCFO-1233xf hydrofluorination). Optionally, HCl is then recovered from the result of the dehydrochlorination reaction. Recovery of HCl is conducted by conventional distillation where it is removed from the distillate. Alternatively, HCl can be recovered or removed by using water or caustic scrubbers. When a water extractor is used, HCl is removed as an aqueous solution. When a caustic solution is used, HCl is removed from system as a chloride salt in aqueous solution. After the recovery or removal of HCl, the organic stream may be sent to a distillation column for separation. HFO-1234yf, collected from the overhead of the column, may be sent to another column for further purification, while a fraction of the mixture of HCFO-1233xf and HCFC-244bb, accumulated in the reboiler, may be sent back to the dehydrochlorination reactor for the recycle of HCFC-244bb, and the rest to the HCFO-1233xf hydrofluorination reactor for the recycle of HCFO-1233xf.

The HFO-1234 so produced is then contacted with at least one amine, in accordance with the present invention, to remove the alkyne impurity such as CF₃C≡CH impurity. In an embodiment, before the removal of the alkyne impurity such as CF₃C≡CH impurity from the HFO-1234, the concentration of the alkyne impurity such as CF₃C≡CH impurity present may be measured using techniques known in the art, such as gas chromatography. Moreover, the amount of impurity present after the addition of amine can also be measured using common techniques, such as gas chromatography.

Many aspects and embodiments have been described above and are merely exemplary and not limiting. After reading this specification, skilled artisans appreciate that other aspects and embodiments are possible without departing from the scope of the invention.

### EXAMPLES

The concepts described herein will be further described in the following examples, which do not limit the scope of the invention described in the claims.

The term "% by GC-MS", as used herein, means the percentage of the peak area measured on the GC-MS spectrum.

The term "% by GC-FID", as used herein, means the percentage of the peak area measured on the GC-FID spectrum.

### Example 1

Example 1 demonstrates that HFO-1234yf can become substantially free of the CF₃C≡CH impurity after contacting with ethylene diamine.

A gaseous HFO-1234yf sample, which was analyzed by GC and GC-MS to contain 0.222 % by GC-FID of the CF₃C≡CH impurity, was bubbled through a scrubber containing about 100 ml ethylene diamine at a flow rate of 16 sccm at room temperature. The effluent gas from the scrubber was analyzed by GC and GC-MS again to show that the concentration of the CF₃C≡CH impurity contained in the HFO-1234yf sample dropped to non-detectable level. The analysis results are also listed in Table 1.

### Example 2

Example 2 demonstrates that HFO-1234yf can become substantially free of the CF₃C≡CH impurity after contacting with morpholine.

A gaseous HFO-1234yf sample, which was analyzed by GC and GC-MS to contain 0.181 % by GC-FID of the CF₃C≡CH impurity, was bubbled through a scrubber containing about 100 ml morpholine at a flow rate of 20 sccm at room temperature. The effluent gas from the scrubber was analyzed by GC and GC-MS again to show that the concentration of the CF₃C≡CH impurity contained in the HFO-1234yf sample dropped to non-detectable level. The analysis results are also listed in Table 1.

### Example 3

Example 3 demonstrates that the concentration of the CF₃C≡CH impurity contained in HFO-1234yf can be largely reduced by contacting with ethanolamine.

A gaseous HFO-1234yf sample, which was analyzed by GC and GC-MS to contain 0.202 % by GC-FID of the CF₃C≡CH impurity, was bubbled through a scrubber containing about 100 ml ethanolamine at a flow rate of 16 sccm at room temperature. The effluent gas from the scrubber was analyzed by GC and GC-MS again to show that the concentration of the CF₃C≡CH impurity contained in the HFO-1234yf sample dropped to 0.049 % by GC-FID. The analysis results are also listed in Table 1.

### Example 4

Example 4 demonstrates that the concentration of the CF₃C≡CH impurity contained in HFO-1234yf can be largely reduced by contacting with triethylamine.

A gaseous HFO-1234yf sample, which was analyzed by GC and GC-MS to contain 0.200 % by GC-FID of the CF₃C≡CH impurity, was bubbled through a scrubber containing about 100 ml triethylamine at a flow rate of 16 sccm at room temperature. The effluent gas from the scrubber was analyzed by GC and GC-MS again to show that the concentration of the CF₃C≡CH impurity contained in the HFO-1234yf sample dropped to 0.095 % by GC-FID. The analysis results are also listed in Table 1.

### Example 5

Example 5 demonstrates that the concentration of the CF₃C≡CH impurity contained in HFO-1234yf can be largely reduced by contacting with pyridine.

A gaseous HFO-1234yf sample, which was analyzed by GC and GC-MS to contain 0.187 % by GC-FID of the CF₃C≡CH impurity, was bubbled through a scrubber containing about 100 ml pyridine at a flow rate of 16 sccm at room temperature. The effluent gas from the scrubber was analyzed by GC and GC-MS again to show that the concentration of the CF₃C≡CH impurity contained in the HFO-1234yf sample dropped to 0.081 % by GC-FID. The analysis results are also listed in Table 1.

**Table 1**

| Example No. | Scrubber | | CF₃C≡CH Concentration (% by GC-FID) | | Flow Rate (sccm) |
|---|---|---|---|---|---|
| | Amine | Length (inch) | Before Scrubbing | After Scrubbing | |
| 1 | ethylene diamine | 2.25 | 0.222 | ND | 16 |
| 2 | morpholine | 2.25 | 0.181 | ND | 20 |
| 3 | ethanolamine | 2.125 | 0.202 | 0.049 | 16 |
| 4 | triethylamine | 2.5 | 0.200 | 0.095 | 16 |
| 5 | pyridine | 2.25 | 0.187 | 0.081 | 16 |

| | | | | | |
|---|---|---|---|---|---|
| ND = non-detectable, which means 2 ppm-molar or less. | | | | | |

### Example 6

Example 6 demonstrates that HCFO-1224yd can become substantially free of the CF₃C≡CCl impurity after contacting with pyridine.

A sealed 240 ml cylinder containing a 148.7g HCFO-1224yd sample with 1.026 % by GC-MS CF₃C≡CCl impurity was cooled to about 0 °C and was charged with 0.5 ml pyridine via a syringe. The cylinder was then warmed up to room temperature and was shaken for 10 minutes. After another two hours, the HCFO-1224yd sample was analyzed by GC-MS to show that the concentration of the CF₃C≡CCl impurity contained in the HCFO-1224yd sample had dropped to non-detectable level.

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification is to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination.

The following represents preferred items of the present invention:
1. A process comprising: contacting a mixture comprising at least one fluoroolefin and at least one R_{f}C≡CX impurity with at least one amine to reduce the concentration of said at least one R_{f}C≡CX impurity in said mixture; wherein R_{f} is a perfluorinated alkyl group, and X is H, F, Cl, Br or I.
2. The process of item 1 wherein the amount of said at least one fluoroolefin in said mixture is at least 90 wt % based on the total weight of said mixture.
3. The process of item 1 wherein said at least one fluoroolefin is selected from the group consisting of CF₃CF=CH₂, CF₃CH=CHF, CF₃CH=CH₂, CF₃CCl=CH₂, CF₃CH=CHCl, CF₃CH=CFCl, CF₃CH=CF₂, CF₃CCl=CHF, CF₃CF=CHF, CF₃CF=CHCl, CF₃CH=CCl₂, CF₃CCl=CHCl, and mixtures thereof, and wherein said at least one R_{f}C≡CX impurity is selected from the group consisting of CF₃C≡CH, CF₃C=CCl, CF₃C≡CF, and mixtures thereof.
4. The process of item 1 wherein said at least one fluoroolefin is selected from the group consisting of CF₃CF=CH₂, CF₃CH=CHF, CF₃CH=CH₂, CF₃CCl=CH₂, CF₃CH=CHCl, CF₃CF=CHCl, and mixtures thereof, and wherein said at least one R_{f}C≡CX impurity is selected from the group consisting of CF₃C≡CH, CF₃C=CCl, CF₃C≡CF, and mixtures thereof.
5. The process of item 1 wherein said at least one fluoroolefin is selected from the group consisting of CF₃CF=CH₂, CF₃CH=CHF, CF₃CCl=CH₂, CF₃CH=CHCl, and mixtures thereof, and wherein said at least one R_{f}C≡CX impurity is selected from the group consisting of CF₃C≡CH, CF₃C=CCl, and mixtures thereof.
6. The process of item 1 wherein said at least one fluoroolefin is CF₃CF=CH₂, and wherein said at least one R_{f}C≡CX impurity is selected from the group consisting of CF₃C≡CH, CF₃C≡CCl, and mixtures thereof.
7. The process of item 1 wherein said at least one fluoroolefin is CF₃CF=CH₂, and wherein said at least one R_{f}C≡CX impurity is CF₃C≡CH.
8. The process of item 1 wherein said at least one fluoroolefin is CF₃CH=CHF, and wherein said at least one R_{f}C≡CX impurity is CF₃C≡CH.
9. The process of item 1 wherein said at least one fluoroolefin is a mixture of CF₃CF=CH₂ and CF₃CH=CHF, and wherein said at least one RₜC≡CX impurity is CF₃C≡CH.
10. The process of item 1 wherein said at least one fluoroolefin is CF₃CH=CHCl, and wherein said at least one R_{f}C≡CX impurity is CF₃C≡CH.
11. The process of item 1 wherein said at least one amine is selected from the group consisting of amine of the formula R₃N, heterocyclic amines, hydrazine and its derivatives, and mixtures thereof, wherein each R is independently a hydrogen, an alkyl group, a heteroalkyl group, an aryl group, or an aralkyl group.
12. The process of item 1 wherein said at least one amine is selected from the group consisting of amine of the formula R₃N, heterocyclic amines, and mixtures thereof, wherein each R is independently a hydrogen, an alkyl group, a heteroalkyl group, or an aralkyl group.
13. The process of item 12 wherein said at least one amine is a polyamine.
14. The process of item 12 wherein said at least one amine is a heterocyclic amine.
15. The process of item 1 wherein said at least one amine is in a solution with a suitable solvent during said contacting step.
16. The process of item 1 wherein the temperature during said contacting step is from about 0 °C to about 60 °C.
17. The process of item 1 wherein the concentration of said at least one R_{f}C≡CX impurity in said mixture is reduced to 200 ppm or less.
18. The process of item 1 wherein the concentration of said at least one R_{f}C≡CX impurity in said mixture is reduced to 50 ppm or less.
19. The process of item 1 wherein the concentration of said at least one R_{f}C≡CX impurity in said mixture is reduced to 2 ppm or less.
20. The process of any one of items 1 to 19 inclusive further comprising recovering said at least one fluoroolefin having reduced concentration of said at least one R_{f}C≡CX impurity.
21. A process for making at least one hydrotetrafluoropropene product selected from the group consisting of CF₃CF=CH₂, CF₃CH=CHF, and mixtures thereof, comprising:
   (a) dehydrohalogenating at least one starting material selected from the group consisting of CF₃CFClCH₃, CF₃CHFCH₂Cl, CF₃CHClCH₂F, CF₃CH₂CHFCl, CF₃CHFCH₂F, CF₃CH₂CF₂H, CF₃CF₂CH₃, and mixtures thereof to produce a product mixture comprising CF₃C≡CH impurity and said at least one hydrotetrafluoropropene product;
   (b) contacting said product mixture with at least one amine to reduce the concentration of said CF₃C≡CH impurity in said product mixture; and
   (c) recovering said at least one hydrotetrafluoropropene product having reduced concentration of said CF₃C≡CH impurity.
22. The process of item 21 wherein said at least one hydrotetrafluoropropene product is CF₃CF=CH₂, and said at least one starting material is selected from the group consisting of CF₃CFClCH₃, CF₃CHFCH₂Cl, CF₃CHFCH₂F, CF₃CF₂CH₃, and mixtures thereof.
23. The process of item 22 wherein said at least one starting material is CF₃CFClCH₃.
24. The process of item 22 wherein said at least one starting material is CF₃CHFCH₂F.
25. The process of item 21 wherein said at least one hydrotetrafluoropropene product is CF₃CH=CHF, and said at least one starting material is selected from the group consisting of CF₃CH₂CHF₂, CF₃CH₂CHFCl, CF₃CHClCH₂F, and mixtures thereof.
26. The process of any one of items 21 to 25 inclusive, wherein the concentration of said CF₃C≡CH impurity in said product mixture is reduced to 200 ppm or less in the contacting step (b).
27. A process for making at least one hydrochlorotrifluoropropene product selected from the group consisting of CF₃CCl=CH₂, CF₃CH=CHCl, and mixtures thereof, comprising:
   (a) dehydrohalogenating at least one starting material selected from the group consisting of CF₃CCl₂CH₃, CF₃CHClCH₂Cl, CF₃CHClCH₂F, CF₃CH₂CHCl₂, CF₃CHFCH₂Cl, CF₃CFClCH₃, CF₃CH₂CHFCl, and mixtures thereof to produce a product mixture comprising CF₃C≡CH impurity and said at least one hydrochlorotrifluoropropene product;
   (b) contacting said product mixture with at least one amine to reduce the concentration of said CF₃C≡CH impurity in said product mixture; and
   (c) recovering said at least one hydrochlorotrifluoropropene product having reduced concentration of said CF₃C≡CH impurity.
28. The process of item 27 wherein at least one hydrochlorotrifluoropropene product is CF₃CH=CHCl, and said at least one starting material is selected from the group consisting of CF₃CHClCH₂Cl, CF₃CH₂CHCl₂, and mixtures thereof.
29. The process of item 27 wherein at least one hydrochlorotrifluoropropene product is CF₃CHCl=CH₂, and said at least one starting material is selected from the group consisting of CF₃CHClCH₂Cl, CF₃CCl₂CH₃, and mixtures thereof.
30. The process of item 27, 28 or 29, wherein the concentration of said CF₃C≡CH impurity in said product mixture is reduced to 200 ppm or less in the contacting step (b).
31. The process of any one of items 21 to 30 inclusive, wherein said at least one amine is selected from the group consisting of amine of the formula R₃N, heterocyclic amines, and mixtures thereof, wherein each R is independently a hydrogen, an alkyl group, a heteroalkyl group, or an aralkyl group.
32. A process for preparing 2,3,3,3-tetrafluoropropene comprising:
   (i) providing a starting composition including a compound of Formulae I, II, or III:

      CX 2=CCl-CH 2X (I);

      CX 3-CCl=CH 2 (11);

      or

      CX 3-CHCl-CH 2X (III)
   wherein X is independently selected from F, Cl, Br, and I, provided that at least one X is not fluorine;
   (ii) contacting the starting composition with a first fluorinating agent to produce a first intermediate composition including 2- chloro-3,3,3-trifluoropropene and a first chlorine- containing byproduct;
   (iii) contacting the first intermediate composition with a second fluorinating agent to produce a second intermediate composition including 2-chloro-1,1,1,2-tetrafluoropropane and 2-chloro-3,3,3-trifluoropropene;
   (iv) dehydrochlorinating at least a portion of the second intermediate composition including 2-chloro-1,1,1,2- tetrafluoropropane to produce a reaction product including 2,3,3,3-tetrafluoropropene and CF₃C≡CH impurity;
   (v) contacting said reaction product produced in step (iv) with at least one amine to reduce the concentration of said CF₃C≡CH impurity in said product mixture and
   (vi) recovering said 2,3,3,3-tetrafluoropropene from said product mixture of step (v), said 2,3,3,3-tetrafluoropropene having reduced concentration of said CF₃C≡CH impurity present relative to that amount originally present in said reaction product of step (iv).

## Claims

1. A composition comprising at least one fluoroolefin, at least one amine and at least one compound of the formula: R_{f}C≡CX, wherein R_{f} is a perfluorinated alkyl group and X is H, F, Cl, Br or I.

2. The composition of claim 1 wherein the amount of the at least one fluoroolefin is at least 90 wt % based on the total weight of the composition.

3. The composition according to claim 1 or 2 wherein the at least one fluoroolefin is CF₃CF=CH₂, CF₃CH=CHF, CF₃CH=CH₂, CF₃CH=CF₂, CF₃CF=CHF, CF₃CCl=CH₂ CF₃CH=CHCl, CF₃CF=CHCl, CF₃CH=CCl₂, CF₃CCl=CHCl, CF₃CH=CFCl, or CF₃CCl=CHF.

4. The composition according to claim 1, 2 or 3 wherein the at least one amine is R1R2R3N, R1NHNH₂, ammonia, polyamine, or heterocyclic amine, wherein each R1, R2, R3 is independently a hydrogen, an alkyl group, an aralkyl group, or a heteroalkyl group, wherein at least one of R1, R2 and R3 is other than hydrogen and wherein each R1, R2 and R3 is independently unsubstituted or substituted with hydroxyl, halogen, alkoxy, and/or amino groups.

5. The composition according to claim 1, 2 or 3 wherein the at least one amine is selected from the group consisting of amine of the formula R₃N, heterocyclic amines, hydrazine and its derivatives, and mixtures thereof, wherein each R is independently a hydrogen, an alkyl group, a heteroalkyl group, an aryl group, or an aralkyl group.

6. The composition according to claim 4 wherein the at least one amine is selected from the group consisting of amine of the formula R₃N, heterocyclic amines, and mixtures thereof, wherein each R is independently a hydrogen, an alkyl group, a heteroalkyl group, or an aralkyl group.

7. The composition of any one of claims 1 to 6 wherein the at least one fluoroolefin is selected from the group consisting of CF₃CF=CH₂, CF₃CH=CHF, CF₃CH=CH₂, CF₃CCl=CH₂, CF₃CH=CHCl, CF₃CF=CHCl, and mixtures thereof, and wherein the at least one compound of the formula RₜC≡CX is selected from the group consisting of CF₃C≡CH, CF₃C≡CCl, CF₃C≡CF, and mixtures thereof.

8. The composition of claim 7 wherein the at least one fluoroolefin is selected from the group consisting of CF₃CF=CH₂, CF₃CH=CHF, CF₃CCl=CH₂, CF₃CH=CHCl, and mixtures thereof, and wherein the at least one compound of the formula RₜC≡CX is selected from the group consisting of CF₃C≡CH, CF₃C≡CCl, and mixtures thereof.

9. The composition of claim 8 wherein the at least one fluoroolefin is CF₃CF=CH₂, and wherein the at least one compound of the formula RₜC≡CX is selected from the group consisting of CF₃C≡CH, CF₃C≡CCl, and mixtures thereof.

10. The composition of any one of claims 1 to 9 wherein the at least one fluoroolefin is CF₃CF=CH₂, and wherein the at least one compound of the formula RₜC≡CX is CF₃C≡CH.

11. The composition of any one of claims 1 to 8 wherein the at least one fluoroolefin is CF₃CH=CHF, and wherein the at least one compound of the formula RₜC≡CX is CF₃C≡CH.

12. The composition of any one of claims 1 to 8 wherein the at least one fluoroolefin is a mixture of CF₃CF=CH₂ and CF₃CH=CHF, and wherein the at least one compound of the formula RₜC≡CX is CF₃C≡CH.

13. The composition of any one of claims 1 to 8 wherein the at least one fluoroolefin is CF₃CH=CHCl, and wherein the at least one compound of the formula RₜC≡CX is CF₃C≡CH.

14. The composition of any one of claims 1 to 13 wherein the at least one amine is a polyamine or a heterocyclic amine.

15. The composition according to any one of claims 1 to 14 wherein the concentration of the compound of the formula R_{f}C≡CX in the composition is 200 ppm or less, more preferably 50 ppm or less, and most preferably 2 ppm or less.
